# EUROPÄISCHE PATENTSCHRIFT

(11) **EP 3 385 953 B1**
(45) Veröffentlichungstag und Bekanntmachung des Hinweises auf die Patenterteilung: **26.08.2020**
(21) Anmeldenummer: 17202491.1
(22) Anmeldetag: 20.11.2017
(51) Int. Cl.: G16H 20/10

(54) **MOBILES DATENGERÄT ZUR MEDIKATIONSÜBERPRÜFUNG**
MOBILE DATA DEVICE FOR MEDICATION CHECKING
APPAREIL INFORMATIQUE MOBILE DESTINÉ AU CONTRÔLE DE LA MÉDICATION

(30) Priorität: 06.04.2017 DE 202017102033 U
(43) Veröffentlichungstag der Anmeldung: 10.10.2018
(73) Patentinhaber: Pietsch, Wolf-Rüdiger, 33161 Hövelhof (DE); Kesselmeier, Manfred, 33098 Paderborn (DE); Hoffmann, Philipp, 33098 Paderborn (DE)
(72) Erfinder: Pietsch, Wolf-Rüdiger, 33161 Hövelhof (DE); Kesselmeier, Manfred, 33098 Paderborn (DE); Hoffmann, Philipp, 33098 Paderborn (DE)
(74) Vertreter: Ostermann, Thomas

(56) Entgegenhaltungen:
- DE-U1-202012 000 410
- US-A1- 2016 161 985
- Anonymous: "My KP Meds", Kaiser Permanente , 30. April 2016 (2016-04-30), XP002781695, Gefunden im Internet: URL:https://mydoctor.kaiserpermanente.org/ mas/mapmg/member_tools/managing_care/MyKPM eds/MyKPMeds.html [gefunden am 2018-06-06]

## Beschreibung

Die Erfindung betrifft ein mobiles Datengerät nach dem Oberbegriff des Patentanspruchs 1.

Aus der DE 297 12 539 U1 ist beispielsweise ein mobiles Datengerät zur Erinnerung von Patienten an die Medikamenteneinnahme bekannt, das in einer Medikamentenspeicher- und Dosiereinrichtung integriert ist. Werden die Medikamente zu einem vorgegebenen Zeitpunkt aus einem Aufnahmefach des mobilen Datengerätes durch den Patienten nicht entnommen, wird optisch bzw. akustisch ein Warnsignal abgegeben, das den Patienten dazu veranlassen soll, die bereitgestellten Medikamente einzunehmen.

Aus der DE 20 2012 000 410 U1 ist ein mobiles Datengerät zur Erinnerung von Patienten an die Einnahme von Medikamenten bekannt, das nicht mit einem Medikamentenspender gekoppelt ist. Das mobile Datengerät verfügt über eine Anzeigeeinheit mit einem Display, mittels dessen medikamenteneinnahmerelevanten Daten dargestellt werden können, wie beispielsweise die Bezeichnung des Medikamentes sowie die einzunehmenden Mengen. Eine Signalgebereinheit sorgt dafür, dass ein entsprechendes Erinnerungssignal abgegeben wird und der Patient veranlasst wird, die in der Anzeigeeinheit dar gestellten medikamenteneinnahmerelevanten Daten zu lesen. Das mobile Datengerät verfügt ferner über eine Eingabeeinheit, mittels derer der Patient die Einnahme des Medikamentes bestätigen kann. Auf diese Weise kann festgestellt werden, wann das betreffende Medikament aufgebraucht ist. Rechtzeitig vor vollständigem Verbrauch des Medikamentes kann der Arzt über eine Schnittstelleneinheit darüber informiert werden, so dass er gegebenenfalls ein neues Rezept ausstellen kann. Durch Weitergabe an eine entsprechende Apotheke kann das Medikament dann rechtzeitig dem Patienten geliefert werden. Ferner umfasst das mobile Datengerät eine Sensoreinheit mit Sensoren zur Ermittlung von körperbezogenen Zustandsdaten, wie beispielsweise Sauerstoffsättigung, Herzfrequenz und dergleichen. Die Schnittstelleneinheit (Kamera) umfasst eine visuelle Kommunikationsschnittstelle, so dass eine Kontaktaufnahme mit einem Arzt zwecks Rückfragen aufgenommen werden kann. Nachteilig an dem mobilen Datengerät ist, dass die Eingabe der korrekten Medikationsdaten nicht sichergestellt ist. Hierbei können Fehler auftreten, die vom behandelnden Arzt möglicherweise erst zu spät erkannt werden.

Aus der Veröffentlichung Anonymous: "My KP Meds", Kaiser Permanente, 30. April 2026 unter https://mydoctor.kaiserpermanente.org/mas/mapmg/member_tools/managing_ care/MyKPMeds/MyKPMeds.html ist ein mobiles Datengerät zur Medikationsüberprüfung bekannt, das eine Speichereinheit mit einem Medikationsplan-Speicher aufweist, in dem von einer autorisierten Instanz erzeugte Medikationsdaten gespeichert sind. Die Medikationsdaten werden zeitabhängig über eine Anzeigeeinheit des mobilen Datengerätes visualisiert, so dass der Nutzer an die Einnahme der Medikation erinnert wird. Die Medikationsdaten werden ausschließlich durch die autorisierte Instanz aktualisiert.

Aus der DE 20 2012 000 410 U1 ist ein mobiles Datengerät zur Erinnerung von Patienten an die Einnahme von Medikamenten bekannt, dass eine Speichereinheit zur Speicherung von Medikationsdaten und eine Anzeigeeinheit zur Darstellung der Medikationsdaten sowie eine Signalgebeeinheit zur Abgabe eines Erinnerungssignals aufweist. Die Speichereinheit weist einen Medikationsplan-Speicher auf, in dem die von einer autorisierten Instanz erzeugten Medikationsdaten gespeichert sind. Ferner umfasst das mobile Datengerät eine Sensoreinheit zur Ermittlung von körperbezogenen Zustandsdaten, die die Sauerstoffsättigung und den Herzschlag des Nutzers betreffen. Bei einem Notfall können dem Arzt somit schnell die vorher gemessenen körperbezogenen Zustandsdaten zur Verfügung gestellt werden. Da das mobile Datengerät eine positive Rückmeldung des Nutzers nach erfolgter Einnahme der Medikation vorsieht, wird die verbrauchte Menge der Medikation dokumentiert. Bei Feststellung der minimalen Restmenge der Medikation wird automatisch von dem mobilen Datengerät eine Anfrage nach neuen Medikamenten an den Arzt oder Apotheker gesendet, so dass der Nutzer den Arzt/Apotheker nicht persönlich aufsuchen muss.

Aus der US 2016/0161985 A1 ist ein mobiles Datengerät zur Erinnerung von Patienten an die Einnahme von Medikamenten und dergleichen bekannt. Das mobile Datengerät weist eine Speichereinheit zur Speicherung der Medikationsdaten, eine Anzeigeeinheit zur Darstellung der Medikationsdaten sowie eine Signalgebereinheit zur Abgabe eines Erinnerungssignals für die Medikamenteneinnahme auf. Ein Medikationsplan-Speicher speichert die Medikationsdaten, die von einer autorisierten Instanz, beispielsweise einem Arzt oder einem Apotheker, stammen. Das mobile Datengerät ermöglicht die zeitabhängige Erinnerung des Nutzers an die Einnahme der Medikamente. Ferner umfasst das mobile Datengerät Sicherungsmittel, so dass der externe Datenaus tausch des Datengerätes ausschließlich mit einer Rechnereinrichtung der autorisierten Instanz erfolgt. Ferner umfasst das mobile Datengerät eine Sensoreinheit zur Ermittlung von körperbezogenen Zustandsdaten. Ferner umfasst das mobile Datengerät einen Körperzustands-Speicher, in dem die von der Sensoreinheit gemessenen körperbezogenen Ist-Zustandsdaten gespeichert sind. Wenn die körperbezogenen Ist-Zustandsdaten, beispielsweise die Herzfrequenz des Nutzers, unter einen vorgegebenen Schwellwert fallen, was eine Lebensbedrohung für den Nutzer nach sich ziehen würde, wird ein Notfallsignal erzeugt, das an den Arzt oder ein Krankenhaus versendet wird.

Aufgabe der vorliegenden Erfindung ist es daher, ein mobiles Datengerät zur Medikationsüberprüfung derart weiterzubilden, dass die Effektivität für die Medikamenteneinnahme von Patienten auf einfache Weise weiter erhöht wird.

Zur Lösung dieser Aufgabe weist die Erfindung die Merkmale des Patentanspruchs 1 auf.

Nach der Erfindung weist das mobile Datengerät einen Medikationsplan-Speicher auf, auf den nur eine autorisierte Instanz, wie beispielsweise ein Arzt oder ein Apotheker, Zugriffsrechte hat. Der Medikationsplan-Speicher weist die Medikationsdaten auf, die dem Patienten in einer Anzeigeeinheit als medikamenteneinnahmerelevante Daten mit der Aufforderung zur Einnahme signalisiert werden. Hierzu weist das mobile Datengerät ein Medikationsplan-Programm auf, das zeitabhängig und/oder ortsabhängig die in dem Medikationsplan-Speicher gespeicherten Medikationsdaten ausliest und der Anzeigeeinheit bzw. Signalgebereinheit zuführt. Der Medikationsplan-Speicher weist die Eigenschaft auf, dass er von jeder Instanz gelesen werden, aber nur von der autorisierten Instanz beschrieben werden kann. Da somit beispielsweise nur der Arzt oder der Apotheker autorisiert sind, den Medikationsplan-Speicher zu beschreiben, können Fehler bei der Eingabe der Medikationsdaten in das mobile Datengerät verhindert werden. Nach der Erfindung weist das mobile Datengerät Sicherungsmittel auf, so dass ein externer Datenaustausch bzw. ein Beschreiben des Medikationsplan-Speicher ausschließlich durch die autorisierte Instanz erfolgen kann. Die autorisierte Instanz ist der einzige Berechtigte zum Schreiben des Medikationsplan-Speichers. Hierdurch ist sichergestellt, dass die Erinnerungsfunktion des mobilen Datengerätes auf korrekten Medikationsdaten beruht. Nach der Erfindung weist das mobile Datengerät einen Körperzustands-Speicher auf, der mit einem Körperkontroll-Programm zusammenwirkt, so dass nach Vergleich der körperbezogenen Ist-Zustandsdaten, die mittels der Sensoreinheit ermittelbar sind, mit körperbezogenen Soll-Zustandsdaten, die in dem Körperzustands-Speicher gespeichert sind, Informationssignale erzeugbar sind, mittels derer der Nutzer über den aktuellen Gesundheitszustand informiert wird. Ist die Abweichung der Ist-Zustandsdaten zu den Soll-Zustandsdaten zu groß, kann alternativ oder zusätzlich auch der Arzt über die Schnittstelleneinheit informiert werden, so dass der Medikationsplan-Speicher zeitnah geändert werden kann.

Nach einer Weiterbildung der Erfindung ist als Sicherungsmittel ein symmetrischer oder unsymmetrischer Authentisierungsalgorithmus vorgesehen, mittels dessen die Kommunikation zwischen der autorisierten Instanz auf der einen Seite und dem mobilen Datengerät auf der anderen Seite gesteuert wird. Es Es erfolgt somit vor Beschreiben des Medikationsplan-Speichers stets eine Überprüfung der Berechtigung des Datensenders. Hierzu kann beispielsweise ein Schlüssel für einen Verschlüsselungsalgorithmus dienen. Bevor die Medikationsdaten in den Medikationsplan-Speicher eingelesen werden können, erfolgt vorzugsweise eine Challenge/Response-Kommunikation zwischen einer Rechnereinrichtung der autorisierten Instanz und dem mobilen Datengerät, wobei beispielsweise das Datengerät der Rechnereinrichtung eine Zufallszahl überträgt, die in der Rechnereinrichtung verschlüsselt wird und dann an das Datengerät zurückgesandt wird. In dem Datengerät erfolgt eine Entschlüsselung dieser gesandten verschlüsselten Zufallszahl und die Überprüfung, ob es sich bei dieser gesandten entschlüsselten Zufallszahl um die ursprünglich gesandte Zufallszahl handelt. Fällt der Vergleich positiv aus, wird die Recheneinheit als autorisierte Instanz angesehen, so dass die entsprechenden Medikationsdaten in dem Medikationsplan-Speicher eingeschrieben werden können. Fällt der Vergleich negativ aus, wird die externe Recheneinheit als nichtautorisierte Instanz angesehen und der Zugriff auf den Medikationsplan-Speicher wird blockiert.. Alternativ kann auch das für die Gesundheitskarte vorgesehene kryptografische Sicherungsverfahren eingesetzt werden, so dass der kryptografische Aufwand hinsichtlich der Rechnereinrichtung des Arztes bzw. der Apotheke verringert wird.

Nach einer Weiterbildung der Erfindung weist das mobile Datengerät eine Eingabeeinheit zur Eingabe eines Bestätigungssignals auf. Wenn das Medikament zu den durch das mobile Datengerät vorgegebenen Zeitpunkten eingenommen worden ist, kann durch Betätigen der Eingabeeinheit durch den Nutzer dies dokumentiert werden. Auf diese Weise hat der Nutzer und gegebenenfalls später der Arzt eine Kontrolle über die korrekte Einnahme des Medikamentes.

Nach einer Weiterbildung der Erfindung weist das mobile Datengerät eine Sensoreinheit zur Ermittlung von körperbezogenen Zustandsdaten, wie beispielweise Puls, Blutdruck und dergleichen auf. Vorteilhaft erfährt das mobile Datengerät hierdurch eine Funktionserweiterung, so dass der Nutzer das mobile Datengerät für mehrere gesundheitsrelevante Aufgaben nutzen kann.

Nach einer Weiterbildung der Erfindung weist die Schnittstelleneinheit eine Kabelverbindung und/oder eine Funkverbindung und/oder eine Kartenleseeinrichtung auf. Die Funkverbindung kann eine LAN- und/oder WLAN-Verbindung sein, die einen aktuellen Informationsstand des Arztes über den Gesundheitszustand bzw. Kontrolle der Medikamenteneinnahme des Nutzers ermöglicht. Insbesondere bei Abweichungen von der vorgesehenen Medikamenteneinnahme bzw. Körperzustandsdaten des Nutzers kann durch Übertragung eines entsprechenden Alarm-/Warnsignals an den Arzt eine schnelle Korrektur bzw. Modifikation des Medikationsplans herbeigeführt werden.

Nach einer Weiterbildung der Erfindung ist das mobile Datengerät mit Haltemittel versehen, so dass eine lösbare Befestigung desselben an einem Körperteil des Nutzers, beispielsweise an einem Arm, erfolgen kann. Beispielsweise kann das Haltemittel als ein Armband ausgebildet sein, so dass das mobile Datengerät als ein Gesundheitsarmband ausgebildet ist.

Ein Ausführungsbeispiel der Erfindung wird nachfolgend anhand der Figur näher erläutert.

Die Figur zeigt:
- Figur: ein Blockschaltbild eines Systems zur Steuerung der korrekten Medikationseinnahme durch einen Nutzer.

Ein System zur Steuerung der Medikationseinnahme bei einem Patienten/Nutzer umfasst im Wesentlichen eine Rechnereinrichtung 1 einer autorisierten Instanz und ein mobiles Datengerät 2, die über eine Kommunikationsschnittstelle 3 miteinander koppelbar sind für einen Datenaustausch.

Die Rechnereinrichtung 1 kann in einer Arztpraxis angeordnet sein, wobei sie aufgrund des exklusiven Zugriffs durch einen Arzt die autorisierte Instanz darstellt. Alternativ kann die Rechnereinrichtung 1 auch in einer Apotheke angeordnet sein, wobei ein Apotheker die autorisierte Instanz bildet.

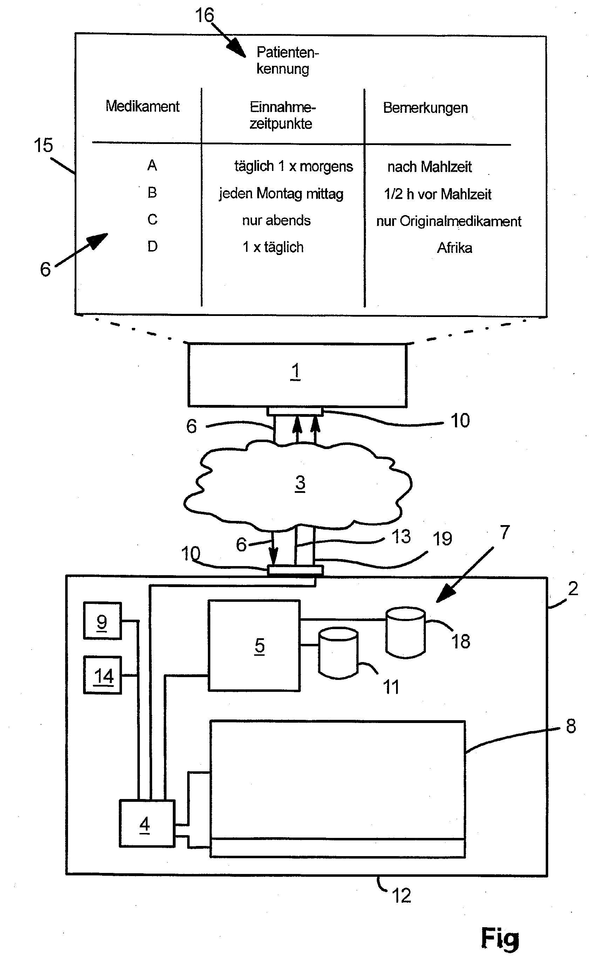

Das mobile Datengerät 2 ist beispielsweise als ein Gesundheitsarmband ausgebildet und kann an einem Körperteil eines Nutzers, beispielsweise an einem Armegelenk desselben, befestigt sein. Das mobile Datengerät 2 weist hierzu als Haltemittel das Armband sowie ein Gehäuse auf, in dem elektrische Bauteilkomponenten gemäß Figur 1 untergebracht sind.

Das mobile Datengerät 2 weist eine Stromversorgungseinheit 4 auf, die eine Batterie oder einen aufladbaren Akku umfassen kann, so dass das mobile Datengerät 2 ortsunabhängig betreibbar ist.

Das mobile Datengerät 2 weist eine Recheneinheit 5 zur Verarbeitung von Medikationsdaten 6, eine Speichereinheit 7 zur Speicherung von Daten, eine Anzeigeeinheit 8 zur Darstellung von medikamenteneinnahmerelevanten Daten bzw. Medikationsdaten auf. Die Recheneinheit 5 umfasst einen Mikroprozessor bzw. Mikrokontroller. Die Speichereinheit 7 weist einen nicht flüchtigen Speicher und gegebenenfalls eine flüchtigen Speicher auf. Die Anzeigeeinheit 8 ist als ein Flüssigkristall-Display ausgebildet, mittels derer einfarbige oder mehrfarbige Informationen darstellbar sind.

Ferner umfasst das mobile Datengerät 2 eine Signalgebereinheit 9 zur Abgabe eines Erinnerungssignals für die Medikationseinnahme auf. Darüber hinaus weist das mobile Datengerät 2 eine Schnittstelleneinheit 10 zum Datenaustausch mit der externen Rechnereinrichtung 1 auf.

Die Speichereinheit 7 weist zum einen einen Medikationsplan-Speicher 11 auf, in dem von der autorisierten Instanz (externen Rechnereinrichtung 1) erzeugten Medikationsdaten 6 abgespeichert sind. Die Speichereinheit 7 umfasst ferner ein Medikationsplan-Programm, dass in der Rechnereinheit 5 ablaufbar ist, so dass zeitabhängig und/oder ortsabhängig die Medikationsdaten 6 aus dem Medikationsplan-Speicher 11 gelesen und über die Anzeigeeinheit 8 dem Nutzer visualisiert und/oder über die Signalgebereinheit 9 dem Nutzer das Erinnerungssignal abgegeben werden kann.

Das mobile Datengerät 2 umfasst ferner eine Eingabeeinheit 12, die über Eingabemittel verfügt, so dass der Nutzer nach Medikamenteneinnahme ein Bestätigungssignal abgeben kann, das in der Speichereinheit 7 gespeichert wird. Gegebenenfalls kann das Bestätigungssignal auch in der Rechnereinheit 5 bzw. in einer hierfür vorgesehenen Steuereinheit weiterverarbeitet werden zur Generierung eines Kontrollsignals 13, dass über die Kommunikationsschnittstelle 3 an die Rechnereinrichtung 1 der autorisierten Instanz abgesendet werden kann. Auf diese Weise ist die autorisierte Instanz (Arzt) darüber informiert, dass die Medikation vorgabegemäß durch den Nutzer eingenommen worden ist. Als Eingabemittel kann beispielsweise eine mechanische Betätigungstaste und/oder eine in dem Flüssigkristall-Display integrierte virtuelle Betätigungstaste ausgebildet sein.

Weiterhin umfasst das mobile Datengerät 2 eine Sensoreinheit 14 zur Ermittlung von körperbezogenen Zustandsdaten. Beispielsweise kann die Sensoreinheit 14 einen Pulsmesser und/oder einen Blutdruckmesser oder dergleichen aufweisen. Die Vornahme der Überprüfung solcher körperbezogenen Zustandsdaten kann genauso durch Vorgabe der autorisierten Instanz vorgeben werden wie die Medikamenteneinnahme. Beispielsweise kann bei einem Herzinfarktpatienten vorgegeben werden, in welchen Abständen er die Herzfrequenz messen soll. Die Anzeigeeinheit 8 bzw. die Signalgebeeinheit 9 erinnert den Nutzer dementsprechend an die Durchführung einer solchen Messung.

Sicherungsmittel sind in dem mobilen Datengerät 2 vorgesehen, so dass ein externer Datenaustausch von Daten ausschließlich mit der autorisierten Instanz bzw. mit der Rechnereinrichtung 1 der autorisierten Instanz erfolgen kann. Zu diesem Zweck weist das mobile Datengerät 2 einen symmetrischen oder unsymmetrischen Authentisierungsalgorithmus auf, so dass unter Anwendung einer Verschlüsselung und/oder eines Entschlüsselungsvorganges festgestellt werden kann, ob das mobile Datengerät 2 mit der autorisierten Rechnereinrichtung 1 kommuniziert. Beispielsweise kann ein Challenge/Response-Verfahren eingesetzt werden, wobei die Rechnereinheit 5 des mobilen Datengerätes 2 eine Zufallszahl erzeugt, die über die Schnittstelleneinheit 10 an die Rechnereinrichtung 1 der autorisierten Instanz übertragen wird. In der Rechnereinrichtung 1 wird diese Zufallszahl mittels eines geheimen Schlüssels verschlüsselt und dann wieder über die Kommunikationsschnittstelle 3 an das mobile Datengerät 2 zurückgesandt. Da das mobile Datengerät 2 den gleichen geheimen Schlüssel aufweist wie die Rechnereinrichtung 1, kann die verschlüsselte Zufallszahl mit dem Schlüssel entschlüsselt werden. Durch anschließenden Vergleich der abgesandten Zufallszahl mit der entschlüsselten zurückgesandten verschlüsselten Zufallszahl kann festgestellt werden, ob die Rechnereinrichtung 1 die erforderliche Autorisierung aufweist. Stimmen die beiden Zufallszahlen überein, handelt es sich um eine autorisierte Rechnereinrichtung 1, so dass ein nachfolgender Datenaustausch erfolgen kann. Stimmen die abgesandte und die empfangende Zufallszahl nicht überein, handelt es sich um eine nicht autorisierte Rechnereinrichtung 1, so dass ein nachfolgender Datenaustausch blockiert wird. Im positiven Fall, also wenn die Rechnereinrichtung 1 als autorisierte Instanz festgestellt worden ist, können in der Rechnereinrichtung 1 die in einem Medikationsplan 15 zusammengefassten Medikationsdaten 6 von der Rechnereinrichtung 1 über die Kommunikationsschnittstelle 3 an das mobile Datengerät 2 übertragen werden.

Der Medikationsplan 15 wurde durch einen Arzt erstellt. Beispielsweise hat er nach Untersuchung eines Patienten demselben mehrere Medikamente A, B, C und D verschrieben. Der Medikationsplan 15 weist beispielsweise als Daten eine Patientenkennung 16 auf, die verschiedenen Medikamente A, B, C, D sowie denselben Medikamenten zugeordnete Einnahmezeitpunkte und gegebenenfalls zusätzliche Informationen. Der Medikationsplan 15 kann eine weitere Spalte aufweisen, in dem Bemerkungen zu den jeweiligen Medikamenten abgelegt sind. Diese Medikationsdaten 6 hat der Arzt beispielsweise über einen PC in das Rechnersystem (Rechnereinrichtung) der Arztpraxis eingegeben. Nach Beendigung der Behandlung beim Arzt kann der Patient beispielsweise an der Empfangstheke der Arztpraxis durch eine Arzthelferin eine Kommunikationsverbindung zwischen der Rechnereinrichtung 1 des Arztes und dem mobilen Datengerät 2 herstellen lassen. Beispielsweise kann das mobile Datengerät 2 mit der Rechnereinrichtung 1 über eine Kabelverbindung verbunden sein. Nach Überprüfung der Authentizität - wie oben beschrieben - kann die Übertragung des Medikationsplans 15 von der Rechnereinrichtung 1 in den Medikationsplan-Speicher 11 erfolgen. Die dem Arzt zur Eingabe des Medikationsplans 15 zur Verfügung gestellte Eingabemaske ist derart ausgebildet, dass die zeit- und/oder ortsabhängigen Eingabedaten in dem mobilen Datengerät 2 so weiter verarbeitet werden, dass sie unter Verknüpfung mit dem entsprechenden Medikament A, B, C, D entsprechende Zeit- und Ortsvorgaben in der Anzeigeeinheit 8 dargestellt werden bzw. die Signalgebeeinheit 9 entsprechend angesteuert wird. Die Rechnereinrichtung 1 weist somit ein Medikationsplan-Eingabeprogramm auf, das die Eingabe nur einer bestimmten Auswahl von fest vorgegebenen Zeit- und/oder Ortsangaben zulässt.

Gegebenenfalls kann das Medikationsplan-Programm derart ausgebildet sein, dass nach Eingabe eines Bestätigungssignals durch den Patienten, welches die Einnahme einer bestimmten Medikation signalisiert, ein Kontrollsignal 13 erzeugt wird, das über die Schnittstelleneinheit 10 bzw. die Kommunikationsschnittstelle 3 an die Rechnereinrichtung 1 an die autorisierte Instanz übertragen wird. Der Arzt hat somit die Möglichkeit, die Einnahme der Medikation zu überprüfen.

Die Schnittstelleneinheit 10 des mobilen Datengerätes 2 kann derart ausgebildet sein, dass die Verbindung zur Rechnereinrichtung 1 der autorisierten Instanz über ein Datennetz, beispielsweise ein LAN- oder WLAN-Netz erfolgen kann. Die Kommunikationsschnittstelle 3 kann somit statt einer Kabelverbindung eine Funkverbindung umfassen. Die Kommunikationsschnittstelle 3 kann beispielsweise als ein Internet- oder als ein mobiles Kommunikationsnetz (GSM) ausgebildet sein. Alternativ kann als Kommunikationsschnittstelle 3 auch eine Kartenleseeinrichtung vorgesehen sein, wobei die Kartenleseeinrichtung vorzugsweise an der Rechnereinrichtung 1 der autorisierten Instanz angeschlossen ist. Das mobile Datengerät 2 weist als Schnittstelleneinheit eine Kartenschnittstelle auf, so dass sie - wie die Gesundheitskarte - in die Kartenleseeinrichtung einsteckbar ist. Zur Authentisierung können die für die Gesundheitskarte vorgesehenen Authentisierungsverfahren eingesetzt werden.

Das mobile Datengerät 2 kann zusätzlich einen Körperzustands-Speicher 18 aufweisen, der mit der Rechnereinheit 5 verbunden ist. In dem Körperzustands-Speicher 18 werden die körperbezogenen Zustandsdaten, die mittels der Sensoreinheit 14 gemessen worden sind, abgespeichert. Der Rechnereinheit 5 kann ein Körperkontroll-Programm zugeordnet sein, das in Abhängigkeit von einem Vergleich der körperbezogenen Ist-Zustandsdaten, die durch die Sensoreinheit ermittelt worden sind, mit körperbezogenen Soll-Zustandsdaten, die beispielsweise über den Medikationsplan 15 von der autorisierten Instanz eingeschrieben worden sind, ein Informationssignal 19 erzeugt wird, das in der Anzeigeeinheit 8 visualisiert wird und/oder über die Schnittstelleneinheit 10 der Rechnereinrichtung 1 der autorisierten Instanz übertragen werden kann. So wird beispielsweise der Arzt beispielsweise über eine erhöhte Herzfrequenz des Patienten informiert, was sich auf die Medikation bzw. Modifikation des Medikationsplanes 15 auswirken kann.

Im Medikationsplan 15 sind beispielsweise Herzmedikamente A, B, C, die zu bestimmten Einnahmezeitpunkten und in Abhängigkeit von Rahmenbedingungen, die unter Bemerkungen festgelegt sind, durch den Patienten eingenommen werden sollen, aufgeführt. Beispielsweise kann als Medikament D Malaria-Tabletten aufgeführt sein, das ortsabhängig, also bei Aufenthalt in Afrika einzunehmen wären.

## Patentansprüche

1. Mobiles Datengerät zur Erinnerung von Patienten an die Einnahme von Medikamenten
- mit einer Stromversorgungseinheit (4),
- mit einer Recheneinheit (5) zur Verarbeitung von Medikationsdaten (6),
- mit einer Speichereinheit (7) zur Speicherung von Medikationsdaten (6),
- mit einer Anzeigeeinheit (8) zur Darstellung von Medikationsdaten (6), mit einer Signalgebereinheit (9) zur Abgabe eines Erinnerungssignals für die Medikamenteneinnahme,
- mit einer Schnittstelleneinheit (10) zum externen Datenaustausch,
- wobei die Speichereinheit (7) einen Medikationsplan-Speicher (11) aufweist, in dem von der autorisierten Instanz erzeugte Medikationsdaten (6) gespeichert sind, und wobei die Speichereinheit (7) ein in der Rechnereinheit (5) ablaufbares Medikationsplan-Programm aufweist, , mittels dessen zeitabhängig die Medikationsdaten (6) aus dem Medikationsplan-Speicher (11) gelesen werden, und über die Anzeigeeinheit (8) visualisiert und/oder über die Signalgebereinheit (9) das Erinnerungssignal abgegeben wird,
- dass Sicherungsmittel vorgesehen sind, derart, dass der externe Datenaustausch ausschließlich mit einer Rechnereinrichtung (1) der autorisierten Instanz erfolgt, wobei die in einem Medikationsplan (15) zusammengefassten Medikationsdaten (6) von der Rechnereinrichtung (1) über eine Kommunikationsschnittstelle (3) an das mobile Datengerät (6) übertragen werden,
- dass eine Sensoreinheit (14) vorgesehen ist zur Ermittlung von körperbezogenen Ist-Zustandsdaten,
- dass ein Körperzustands-Speicher (18) vorgesehen ist, in dem die von der mittels der Sensoreinheit (14) gemessenen körperbezogenen Ist-Zustandsdaten gespeichert sind, wobei der Körperzustands-Speicher (18) mit der Rechnereinheit (5) verbunden ist,
**dadurch gekennzeichnet,**
- **dass** der Rechnereinheit (5) ein Körperkontroll-Programm zugeordnet ist, das in Abhängigkeit von einem Vergleich der körperbezogenen Ist-Zustandsdaten, die durch die Sensoreinheit (14) ermittelt werden, mit körperbezogenen Soll-Zustandsdaten, die über den Medikationsplan (15) von der autorisierten Instanz in den Körperzustands-Speicher (18) eingeschrieben sind, ein Informationssignal (19) erzeugt,
- **dass** das Informationssignal (19) als Alarm-/Warnsignal über die Schnittstelleneinheit (10) der Rechnereinheit (1) der autorisierten Instanz übertragen wird, wenn die Abweichung der Ist-Zustandsdaten zu den Soll-Zustandsdaten zu groß ist, so dass von der autorisierten Instanz ein geänderter Medikationsplan (15) über die Kommunikationsschnittstelle (3) an das mobile Datengerät (2) übertragen werden kann, wobei der Medikationsplan-Speicher (11) geändert wird.

2. Mobiles Datengerät nach Anspruch 1, **dadurch gekennzeichnet, dass** als Sicherungsmittel ein symmetrischer oder unsymmetrischer Authentisierungsalgorithmus vorgesehen ist, wobei zur Freigabe des Beschreibens des Medikationsplan-Speichers (11) eine Authentisierungsroutine unter Verwendung eines Schlüssels erfolgt.

3. Mobiles Datengerät nach Anspruch 1 oder 2, **dadurch gekennzeichnet, dass** eine Eingabeeinheit (12) mit Eingabemitteln zur Eingabe eines Bestätigungssignals des Patienten vorgesehen ist, das in der Recheneinheit weiterverarbeitbar ist zur Erzeugung eines Kontrollsignals (13), das über die Schnittstelleneinheit (10) an die autorisierte Instanz absendbar ist.

4. Mobiles Datengerät nach einem der Ansprüche 1 bis 3, **dadurch gekennzeichnet, dass** die Eingabemittel eine mechanische Betätigungstaste und/oder ein in der Anzeigeeinheit (8) integrierte virtuelle Betätigungstaste umfasst.

5. Mobiles Datengerät nach einem der Ansprüche 1 bis 4, **dadurch gekennzeichnet, dass** die Schnittstelleneinheit (10) derart ausgebildet ist, dass eine Verbindung zwischen dem mobilen Datengerät (2) und einer Rechnereinrichtung (1) der autorisierten Instanz über eine Kabelverbindung und/oder eine Funkverbindung und/oder eine Kartenleseeinrichtung erfolgt.

6. Mobiles Datengerät nach einem der Ansprüche 1 bis 5, **dadurch gekennzeichnet, dass** das mobile Datengerät (2) Haltemittel umfasst zur lösbaren Befestigung an einem Körperteil des Patienten.

7. System zur Steuerung der Medikamenteneinnahme mit einem mobilen Datengerät (2) nach einem der Ansprüche 1 bis 6 und mit einer Rechnereinrichtung (1) der autorisierten Instanz, wobei in der Rechnereinrichtung (1) eine Medikationseingabemaske zur Eingabe des Medikationsplans und/oder ein Medikationsplan-Eingabeprogramm und ein Schlüssel zur Autorisierung gegenüber dem mobilen Datengerät integriert sind

## Claims

1. A mobile data device for reminding patients to take medications, comprising
- a power supply unit (4),
- a computing unit (5) for processing medication data (6),
- a memory unit (7) for storing medication data (6),
- a display unit (8) for displaying medication data (6),
- a signaling unit (9) for emitting a reminder signal for the taking of medications,
- an interface element (10) for external data exchange,
- wherein the memory unit (7) has a medication plan memory (11) in which the medication data (6) generated by the authorized entity are stored, and wherein the memory unit (7) has a medication program able to be run in the computing unit (5) by means of which the medication data (6) are time-dependently read from the medication plan memory (11) and visualized via the display unit (8) and/or the reminder signal is emitted via the signaling unit (9),
- security means are provided such that the external data exchange takes place exclusively with a computer (1) of the authorized entity, wherein the medication data (6) summarized in a medication plan (15) are transmitted by the computer (1) to the mobile data device (2) via a communication interface (3),
- a sensor unit (14) is provided for detecting body-related status data,
- a body-status memory (18) is provided in which the current status data measured by means of the sensor unit (14) are stored, wherein the body-status memory (18) is connected to the computing unit (5),
**characterized in that**
- a body-control program is assigned to the computing unit (5), said program generating an information signal (19) depending on a comparison of the body-related current status data detected by the sensor unit (14) with body-related target status data entered into the body-status memory (18) by the authorized entity via the medication plan (15),
- the information signal (19) is transmitted as an alarm/warning signal via the interface unit (10) of the computer (1) of the authorized entity when the deviation of the current status data from the target status data is too great, so that an amended medication plan (15) can be transmitted from the authorized entity to the mobile data device (2) via the communication interface (3), wherein the medication plan memory (11) is amended.

2. The mobile data device according to Claim 1, **characterized in that** a symmetrical or asymmetrical authentication algorithm is provided as a security means, wherein an authentication routine is carried out using a key in order to disclose the description of the medication plan memory (11).

3. The mobile data device according to Claim 1 or 2, **characterized in that** an input unit (12) with input means for inputting a confirmation signal of the patient is provided, said confirmation signal being further processable in the computing unit to generate a control signal (13) which is able to be sent to the authorized entity via the interface unit (10).

4. The mobile data device according to any one of claims 1 to 3, **characterized in that** the input means comprises a mechanical actuating button and/or a virtual actuating button integrated into the display unit (8).

5. The mobile data device according to any one of claims 1 to 4, **characterized in that** the interface unit (10) is configured such that a connection between the mobile data device (2) and a computer (1) of the authorized entity is established via a cable connection and/or a radio connection and/or a card-reading apparatus.

6. The mobile data device according to any one of claims 1 to 5, **characterized in that** the mobile data device (2) comprises holding means for releasable attachment to a body part of the patient.

7. A system for controlling the taking of medication with a mobile data device (2) according to any one of claims 1 to 6 and with a computer (1) of the authorized entity, wherein a medication input mask for inputting the medication plan and/or a medication plan input program and a key for authentication in relation to the mobile data device are integrated into the computer (1).

## Revendications

1. Appareil informatique mobile pour rappeler à des patients la prise de médicaments,
- avec une unité d'alimentation en courant (4),
- avec une unité informatique (5) pour le traitement de données de médication (6),
- avec une unité de mémorisation (7) pour la mémorisation de données de médication (6),
- avec une unité d'affichage (8) pour la représentation de données de médication (6), avec une unité d'émission de signaux (9) pour l'émission d'un signal de rappel de la prise de médicaments,
- avec une unité d'interface (10) pour l'échange externe de données,
- sachant que l'unité de mémorisation (7) présente une mémoire de plan de médication (11), dans laquelle des données de médication (6), générées par l'instance autorisée, sont mémorisées, et sachant que l'unité de mémorisation (7) présente un programme de plan de médication pouvant se dérouler dans l'unité informatique (5), au moyen duquel les données de médication (6) peuvent être lues à partir de la mémoire de plan de médication (11) en fonction du temps, et ou visualisées par l'intermédiaire de l'unité d'affichage (8) et / ou que le signal de rappel peut être émis par l'intermédiaire de l'unité d'émission de signaux (9),
- que des moyens de sécurité sont prévus de sorte que l'échange externe de données s'effectue exclusivement avec l'installation informatique (1) de l'instance autorisée, sachant que les données de médication (6), groupées dans un plan de médication (15), sont transmises de l'installation informatique (1) à l'appareil informatique mobile (2) par l'intermédiaire d'une interface de communication (3),
- qu'une unité de captage (14) est prévue pour le captage concernant l'état physique réel,
- qu'une mémoire d'état physique (18) est prévue, dans laquelle sont mémorisées les données d'état physique réel, mesurées au moyen de l'unité de captage (14), sachant que la mémoire d'état physique (18) est reliée à l'unité informatique (5),
**caractérisé en ce que**,
- à l'unité informatique (5), est associé un programme de contrôle physique, qui génère un signal d'information (19), en fonction d'une comparaison établie entre les données d'état physique réel, captées par l'unité de captage (14), et les données d'état physiques de consigne, indiquées dans la mémoire d'état physique (18) en fonction du plan de médication (15) de l'instance autorisée,
- que le signal d'information (19) est transmis, en tant que signal d'alarme, à l'instance autorisée, par l'intermédiaire de l'unité d'interface (10) de l'unité informatique (1), lorsque la divergence entre les données d'état réel et les données d'état de consigne est trop grande, de sorte que l'instance autorisée puisse, par l'intermédiaire de l'interface de communication (3), transmettre à l'appareil informatique mobile (2) un plan de médication (15) modifié, sachant que la mémoire de plan de médication (11) est modifiée.

2. Appareil informatique mobile selon la revendication 1, **caractérisé en ce que**, comme moyen de sécurité, un algorithme d'authentification symétrique ou asymétrique est prévu, sachant que, pour la libération de la description de la mémoire de plan de médication (11), une routine d'authentification est effectuée en utilisant une clé.

3. Appareil informatique mobile selon revendication 1 ou 2, **caractérisé en ce qu'**est prévue une unité d'entrée (12) comprenant des moyens d'entrée pour l'entrée d'un signal de confirmation du patient qui peut être traité dans l'unité informatique pour générer un signal de contrôle (13), qui, par l'intermédiaire de l'unité d'interface (10), peut être envoyé à l'instance autorisée.

4. Appareil informatique mobile selon l'une des revendications 1 à 3, **caractérisé en ce que** les moyens d'entrée comprennent une touche d'actionnement mécanique et / ou une touche d'actionnement virtuelle, intégrée dans l'unité d'affichage (8).

5. Appareil informatique mobile selon l'une des revendications 1 à 4, **caractérisé en ce que** l'unité d'interface (10) est conçue de sorte que, par l'intermédiaire d'un câble et / ou par une liaison radio et / ou une installation de lecture de cartes, une communication est établie entre l'appareil informatique mobile (2) et une installation informatique (1) de l'instance autorisée.

6. Appareil informatique mobile selon l'une des revendications 1 à 5, **caractérisé en ce que** l'appareil informatique mobile (2) comprend des moyens de maintien pour la fixation amovible sur une partie du corps d'un patient.

7. Système de commande de la prise de médicaments avec un appareil informatique mobile (2) selon l'une des revendications 1 à 6 et avec une installation informatique (1) de l'instance autorisée, sachant que, dans l'installation informatique (1), sont intégrés un masque d'entrée de médication pour le plan de médication et / ou un programme de plan de médication-plan d'entrée et une clé pour l'autorisation vis-à-vis de l'appareil informatique mobile.
